# NEUE EUROPÄISCHE PATENTSCHRIFT

(11) **EP 3 515 583 B2**
(45) Veröffentlichungstag und Bekanntmachung des Hinweises auf die Entscheidung über den Einspruch: **20.05.2026**
(45) Hinweis auf die Patenterteilung: 08.07.2020
(21) Anmeldenummer: 17743313.3
(22) Anmeldetag: 19.07.2017
(51) Int. Cl.: B01J 2/16, A61K 9/16

(54) **WIRBELSCHICHTANLAGE**
FLUIDISED BED SYSTEM
INSTALLATION À LIT FLUIDISÉ

(30) Priorität: 21.09.2016 DE 102016218085
(43) Veröffentlichungstag der Anmeldung: 31.07.2019
(73) Patentinhaber: Hüttlin GmbH, 79650 Schopfheim (DE)
(72) Erfinder: MICHAELIS, Marc, 79539 Loerrach (DE); WOLPENSINGER, Bernd, 79618 Rheinfelden (DE); BOERNER, Matthias, 79650 Schopfheim (DE); ZERRER, Norbert, 79650 Schopfheim (DE); PAASCHE, Christian Karl, 79713 Bad Saeckingen (DE)
(74) Vertreter: DREISS Patentanwälte PartG mbB
(86) Internationale Anmeldenummer: PCT/EP2017/068259
(87) Internationale Veröffentlichungsnummer: WO 2018/054576

(56) Entgegenhaltungen:
- EP-B2- 0 332 929
- WO-A1-03/033126
- WO-A1-2016/090493
- WO-A1-2017/211501
- CH-A5- 686 343
- JP-A- 2004 290 741
- US-A1- 2003 000 228
- US-A1- 2003 012 873
- US-B1- 6 187 076
- UHLEMANN, HANS: "Wirbelschicht-Sprühgranulation", VDI-BUCH, 2000, Berlin ; Heidelberg ; New York ; Barcelona; Hongkong ; London ; Mailand ; Paris; Singapur ; Tokio : Springer, pages 1 - 628, ISBN: 3-540-66985-X

## Beschreibung

### Stand der Technik

Die Erfindung betrifft eine Wirbelschichtanlage. Insbesondere betrifft die Erfindung eine Wirbelschicht-Granulationsanlage. Mit der Wirbelschichtanlage beziehungsweise der Wirbelschicht-Granulationsanlage sind insbesondere pharmazeutische Pulver bearbeitbar.

Aus dem Stand der Technik ist die Verarbeitung von pharmazeutischen Pulvern in der Wirbelschicht bekannt. Hierzu zählen insbesondere die Prozesse trocknen, coaten und granulieren. Für kontinuierlich betriebene pharmazeutische Nassgranulationseinheiten werden derzeit vorzugsweise Systeme mit Extruder und nachgeschalteten Wirbelschichttrocknern eingesetzt. Nachteilig sind dabei die veränderten Granulateigenschaften durch die höhere Kompaktierung im Extruder. Die Feuchtgranulation pharmazeutischer Pulver in der Wirbelschichtanlage ist von batchweise betriebenen Systemen bekannt. Allerdings können diese Systeme bisher nicht zufriedenstellend, vollautomatisch, in kontinuierlicher oder semikontinuierlicher Fahrweise parallel betrieben werden. Insbesondere ist keine Lösung in der pharmazeutischen Produktion bekannt oder akzeptiert, die durchgängige Granulation eines solchen Systems und automatisierte oder teilautomatisierte Zwischenreinigungen von einzelnen Granuliereinheiten gewährleistet. Aus der Chemieproduktion und/oder Lebensmittelproduktion sind kontinuierlich arbeitende Wirbelschicht-Granulierapparate bekannt, welche als Rinne mit oder ohne Wehre, oder mit klassierendem oder nicht klassierendem Austrag konstruiert sind. Diese Systeme werden jedoch sowohl in der pharmazeutischen Industrie als auch von den Zulassungsbehörden nicht akzeptiert. Zusätzlich gibt es bisher in der pharmazeutischen Industrie keine adäquate Prozessregelung und Einbindung einer übergeordneten Gesamtsteuerung der Wirbelschicht-Granulationsanlage in eine gesamte Produktionslinie, sowie die Möglichkeit kontinuierlich oder semikontinuierlich in paralleler Fahrweise zu granulieren und zu beschichten (coaten).

Beispiele für Wirbelschichttrocknungsanlagen mit mehreren Wirbelschichtkammern sind in der US 7 908 765 B2, in der DE 10 2013 102 133 A1 sowie in der DE 10 2014 103 661 A1 gezeigt. Weiterer gattungsgemässer Stand der Technik ist ausserdem aus der WO 03/033126 A1 bekannt.

### Offenbarung der Erfindung

Die Erfindung betrifft eine Wirbelschichtanlage mit den Merkmalen des Anspruchs 1 und ein Verfahren mit den Merkmalen des Anspruchs 10. Die erfindungsgemäße Wirbelschichtanlage erlaubt ein vollautomatisches Granulieren und Trocknen von pharmazeutischen Pulvern. Die erfindungsgemäße Wirbelschichtanlage umfasst eine Vielzahl von funktional parallel angeordneten Granuliereinheiten. Dabei ist jede Granuliereinheit einzeln über einen Einlass zu beladen und über einen Auslass zu entleeren. Bevorzugt weist die Granuliereinheit eine Öffnung auf, über die der Einlass und der Auslass realisiert sind. Jede Granuliereinheit weist dazu einen Wirbelschichtbehälter auf, sowie einen Einlass und einen Auslass, die jeweils an dem Wirbelschichtbehälter angeordnet sind. Außerdem weist jede Granuliereinheit eine Fluidzufuhr sowie eine Fluidabfuhr auf, die ebenfalls jeweils an dem Wirbelschichtbehälter angeordnet sind. Über die Fluidzufuhr ist ein Arbeitsfluid, insbesondere Luft, in den Wirbelschichtbehälter einbringbar, wo sich das Fluid, insbesondere die Arbeitsluft, mit einem über den Einlass eingebbaren pulverförmigen Feststoff vermischt. Über die Fluidabfuhr ist das Fluid aus dem Wirbelschichtbehälter entnehmbar, während das Pulver oder das fertige Produkt über den Auslass aus dem Wirbelschichtbehälter entnehmbar ist. Weiterhin weist die Granuliereinheit zumindest eine Einspritzdüse zum Einspritzen eines Bearbeitungsstoffs in den Wirbelschichtbehälter auf. Somit lässt sich das innerhalb des Wirbelschichtbehälters befindliche Pulver mit dem Bearbeitungsstoff coaten oder granulieren. Dabei ist vorgesehen, dass eine Steuereinheit vorgesehen ist, die zum Einstellen von Prozessbedingungen innerhalb jeder Granuliereinheit ausgebildet ist. Die Prozessbedingungen lassen sich vorteilhafterweise durch Parameter, wie insbesondere Temperatur, Druck oder Feuchtigkeit, des über den Fluidzufuhr zugeführten Fluids einstellen. Außerdem lassen sich die Prozessbedingungen vorteilhafterweise durch eine Menge des zugeführten Fluids und/oder durch eine Menge des über den Einlass zugeführten Pulvers oder pulverförmigen Feststoffs einstellen. Das Einstellen der Prozessbedingungen innerhalb jeder Granuliereinheit erfolgt dabei vorteilhafterweise unabhängig von allen anderen Granuliereinheiten. Durch einen derart automatisierten, parallelen und auf die einzelnen Granuliereinheiten abgestimmten Betrieb kann in einem regelmäßigen Zeitintervall Granulat erzeugt werden.

Die Wirbelschichtbehälter sind vorteilhafterweise zylinderförmig aufgebaut und die Wirbelschichtbehälter umfassen einen Deckel sowie einen Boden. Zwischen dem Deckel und dem Boden erstreckt sich eine Seitenwand. Dabei ist vorgesehen, dass die Fluidzufuhr in dem Boden und die Fluidabfuhr in dem Deckel angeordnet ist, während der Einlass und der Auslass in der Seitenfläche angeordnet sind. Auf diese Weise kann ein über den Einlass in den Wirbelschichtbehälter eingebrachter pulverförmige Feststoff mit dem Arbeitsfluid, das über den Fluideinlass in den Wirbelschichtbehälter eingebracht wird und das innerhalb des Wirbelschichtbehälters zu dem Fluidauslass strömt, vermischt werden, wodurch der zu bearbeitende pulverförmige Feststoff einen fluidähnlichen Zustand einnimmt. Die Seitenfläche weist eine Öffnung auf, über die Einlass und Auslass realisiert sind.

Die Unteransprüche haben bevorzugte Weiterbildungen der Erfindung zum Inhalt.

Vorteilhafterweise ist vorgesehen, dass die Wirbelschichtanlage eine Einlassleitung aufweist. Die Einlassleitung verbindet alle Einlässe aller Granuliereinheiten. Die Einlassleitung kann auch in ein zentrales Verteilsystem münden. Besonders vorteilhaft ist vorgesehen, dass die Granuliereinheiten ringförmig angeordnet sind, sodass die Einlassleitung eine Ringleitung darstellt. Alternativ sind die Granuliereinheiten bevorzugt linear in zumindest einer Reihe angeordnet, sodass die Einlassleitung ebenfalls linear verläuft. Vorteilhafterweise ist jeder Einlass über ein Ventil mit der Einlassleitung verbunden, sodass jeder Wirbelschichtbehälter jeder Granuliereinheit separat über die Einlassleitung befüllt werden kann. Besonders vorteilhaft ist vorgesehen, dass genau eine, insbesondere ringförmige, Einlassleitung vorhanden ist. Alternativ kann die Einlassleitung bevorzugt auch für jede Granuliereinheit eine einzelne Leitung umfassen. Die Einlassleitung ist außerdem bevorzugt mit einer Abfallsammelvorrichtung verbunden, über die überschüssiges und / oder fehlerhaftes Material aufgefangen werden kann. Alternativ kann jede einzelne Leitung direkt ohne Ventil mit dem Einlass einer Abfallsammelvorrichtung verbunden sein.

Weiterhin ist bevorzugt vorgesehen, dass die Wirbelschichtanlage eine Auslassleitung aufweist. Über die Auslassleitung sind alle Auslässe aller Granuliereinheiten verbunden. Wiederum ist bevorzugt vorgesehen, dass die Granuliereinheiten ringförmig angeordnet sind, sodass die Auslassleitung eine Ringleitung ist. Die Anordnung kann alternativ auch linear sein, sodass die Auslassleitung ebenfalls linear verläuft. Jeder Auslass ist vorteilhafterweise über ein eigenes Ventil mit der Auslassleitung verbunden, sodass wahlweise einzelne Granuliereinheiten entleert werden können. Auch die Auslassleitung ist vorteilhafterweise mit einer Abfallsammelvorrichtung verbunden. So ist über die Abfallsammelvorrichtung überschüssiges Material auffangbar und bevorzugt entsorgbar. Die Abfallsammelvorrichtung kann dieselbe Abfallsammelvorrichtung wie zuvor beschrieben sein. Sowohl für die Einlassleitung als auch für die Auslassleitung ist besonders vorteilhaft vorgesehen, dass diese ringförmig sind, wobei die Leitungen auch andere Formen aufweisen können, insbesondere linear verlaufen können. Dabei können die Einlassleitung und/oder die Auslassleitung auch ringsegmentförmig ausgebildet sein. In einer weiteren besonders bevorzugten Ausführungsform sind die Granuliereinheiten ringförmig angeordnet, wobei die Einlassleitung die Granuliereinheiten von außen, zumindest teilweise, umschließt, während die Auslassleitung, zumindest ringsegmentförmig, innerhalb der ringförmig angeordneten Granuliereinheiten angeordnet ist. Somit ist ein sehr platzsparender Aufbau gewährleistet. Durch eine lineare Anordnung der Granuliereinheiten ist eine gute Zugänglichkeit jeder einzelnen Granuliereinheit innerhalb der Wirbelschichtanlage erreichbar. Somit ist insbesondere auch eine einfache manuelle Reinigbarkeit erreichbar.

In einer vorteilhaften Ausführungsform umfasst die Wirbelschichtanlage ein Reinigungsmodul. Das Reinigungsmodul ist mit jeder Granuliereinheit wirkverbunden. Besonders vorteilhaft ist eine ringförmige oder ringsegmentförmige Reinigungsleitung vorhanden, die die Wirbelschichtbehälter aller Granuliereinheiten verbindet. Somit ist jede Granuliereinheit von dem Reinigungsmodul reinigbar. Wiederum ist besonders vorteilhaft vorgesehen, dass Ventile vorhanden sind, um jeden Wirbelschichtbehälter mit der Reinigungsleitung zu verbinden. Somit ist sichergestellt, dass eine Reinigung einer Granuliereinheit unabhängig von anderen Granuliereinheiten erfolgen kann. Dies ermöglicht insbesondere auch, dass eine Granuliereinheit gereinigt wird, während andere Granuliereinheiten weiterhin im Betrieb sind.

Besonders vorteilhaft ist das Reinigungsmodul zum Durchführen eines Reinigens mit manuellen Nacharbeiten ausgebildet. Ein solches Reinigen wird auch Wipe In Place (WIP) genannt. Alternativ oder zusätzlich ist das Reinigungsmodul vorteilhafterweise ausgebildet, ein Reinigen ohne manuelles Nacharbeiten durchzuführen. Ein solches Reinigen wird auch Clean in Place (CIP) genannt. Die Wirbelschichtanlage ist durch das Reinigungsmodul somit zumindest teilautomatisiert reinigbar, wobei die Reinigung nur einzelne der Vielzahl von Granuliereinheiten betreffen kann. Somit wird eine Produktion mittels der Wirbelschichtanlage durch die Reinigung nicht oder nur gering beeinträchtigt.

Bevorzugt weist die Wirbelschichtanlage zumindest einen Moduleinlass auf. Der Moduleinlass ist mit den Einlässen aller Granuliereinheiten verbunden. Besonders vorteilhaft ist die zuvor beschriebene Einlassleitung vorhanden, sodass der Moduleinlass mit der Einlassleitung verbunden ist. Der Moduleinlass ist von der Steuereinheit ansteuerbar. Somit ist die Steuereinheit eine zentrale Instanz nicht nur zum Ansteuern der einzelnen Granuliereinheiten, sondern auch zum Ansteuern des Moduleinlasses. Daher lässt sich der Granulierprozess innerhalb der Granuliereinheiten sicher und zuverlässig durchführen, da sämtliche Vorarbeitsparameter bekannt sind.

Bei dem Moduleinlass kann es sich insbesondere um ein zentrales Verteilungsmodul und/oder eine Weiche und/oder eine Ringleitung und/oder ein Umschaltventil und oder einen Pufferbehälter u handeln. Somit lässt sich stets die optimale Menge, die für eine der Granuliereinheiten benötigt wird, bereitstellen und der Granuliereinheit zuführen. Alternativ oder zusätzlich kann das Vorbereitungsmodul eine Eingangsmühle und/oder eine Eingangswaage umfassen.

Weiterhin ist bevorzugt vorgesehen, dass die Wirbelschichtanlage zumindest einen Modulauslass aufweist. Der Modulauslass ist mit den Auslässen aller Granuliereinheiten verbunden. Besonders vorteilhaft ist die zuvor beschriebene Auslassleitung vorhanden, wobei der Modulauslass mit der Auslassleitung verbunden ist. Der Modulauslass ist von der Steuereinheit ansteuerbar. Schließlich ist bevorzugt vorgesehen, dass die Einspritzdüse in einem Boden und/oder in einem Deckel und/oder in einer Seitenwand der Granuliereinheit angeordnet ist. Besonders vorteilhaft sind mehrere Einspritzdüsen vorhanden. Über die Einspritzdüsen ist ein Bearbeitungsstoff, insbesondere eine Granulierflüssigkeit, in den Wirbelschichtbehälter einspritzbar, sodass das sich innerhalb des Wirbelschichtbehälters befindliche Pulver granuliert oder anderweitig verarbeitet, insbesondere durch coaten, werden kann. Somit ist insbesondere die Herstellung pharmazeutischer Mittel ermöglicht. Besonders vorteilhaft können mehrere Einspritzdüsen, insbesondere an unterschiedlichen Orten innerhalb der Granuliereinheit und/oder des Wirbelschichtbehälters, angeordnet sein.

Die Erfindung betrifft außerdem ein Verfahren zur semi-kontinuierlichen Herstellung von, insbesondere pharmazeutischen, Granulaten innerhalb einer Wirbelschichtanlage. Dabei ist vorgesehen, dass einzelne Granuliereinheiten der Wirbelschichtanlage einzelnen und/oder unabhängig voneinander betreibbar sind. Außerdem ist bevorzugt vorgesehen, dass die einzelnen Granuliereinheiten unabhängig von einem Betrieb der anderen Granuliereinheiten reinigbar sind. Auf diese Weise ist ein unterbrechungsfreier Betrieb der Wirbelschichtanlagen durchführbar.

### Kurze Beschreibung der Zeichnung(en)

Nachfolgend werden Ausführungsbeispiele der Erfindung unter Bezugnahme auf die begleitende Zeichnung im Detail beschrieben. In der Zeichnung ist:
- Figur 1: eine schematische Abbildung einer Granuliereinheit einer Wirbelschichtanlage gemäß einem Ausführungsbeispiel der Erfindung,
- Figur 2: eine schematische Abbildung einer alternativen Granuliereinheit einer Wirbelschichtanlage gemäß dem Ausführungsbeispiel der Erfindung,
- Figur 3: eine schematische Abbildung einer Wirbelschichtanlage gemäß dem Ausführungsbeispiel der Erfindung in einer zweiten Alternative,
- Figur 4: eine erste Alternative eines Moduleinlasses einer Wirbelschichtanlage gemäß dem Ausführungsbeispiel der Erfindung,
- Figur 5: eine zweite Alternative eines Moduleinlasses einer Wirbelschichtanlage gemäß dem Ausführungsbeispiel der Erfindung,
- Figur 6: eine dritte Alternative eines Moduleinlasses einer Wirbelschichtanlage gemäß dem Ausführungsbeispiel der Erfindung,
- Figur 7: Alternativen eines Modulauslasses einer Wirbelschichtanlage gemäß dem Ausführungsbeispiel der Erfindung,
- Figur 8: eine erste alternative Anordnung der Granuliereinheiten der Wirbelschichtanlage gemäß dem Ausführungsbeispiel der Erfindung,
- Figur 9: eine zweite alternative Anordnung der Granuliereinheiten der Wirbelschichtanlage gemäß dem Ausführungsbeispiel der Erfindung,
- Figur 10: eine dritte alternative Anordnung der Granuliereinheiten der Wirbelschichtanlage gemäß dem Ausführungsbeispiel der Erfindung, und
- Figur 11: eine weitere schematische Abbildung eines Teils der Wirbelschichtanlage gemäß dem Ausführungsbeispiel der Erfindung.

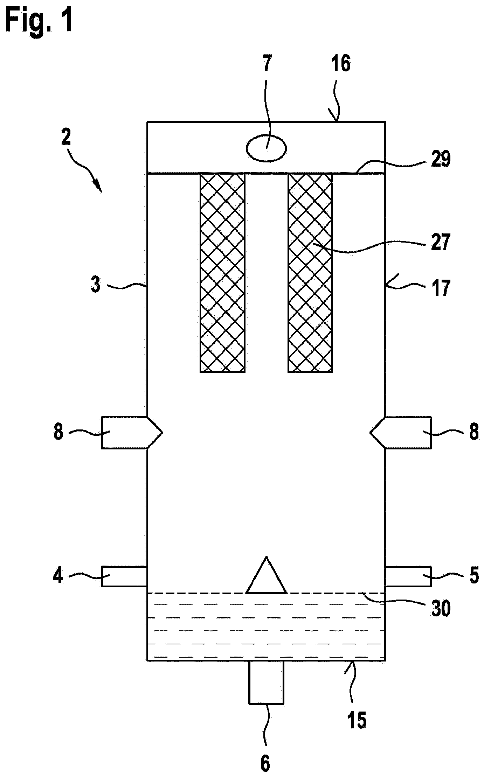

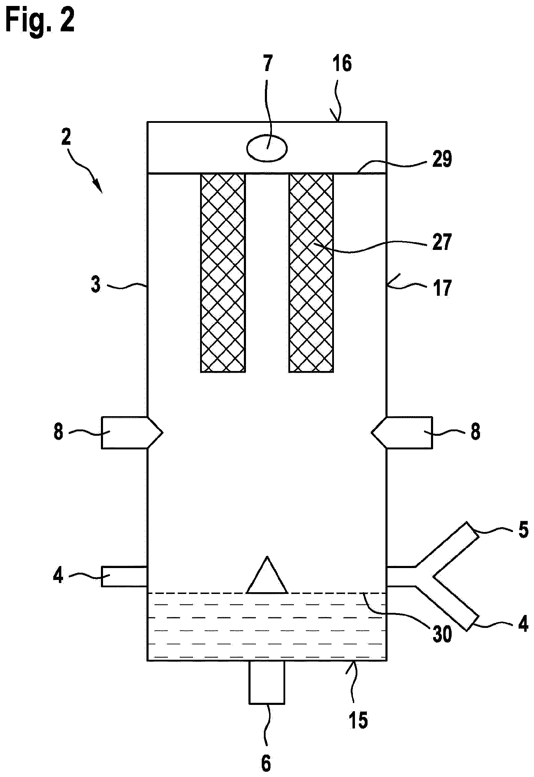

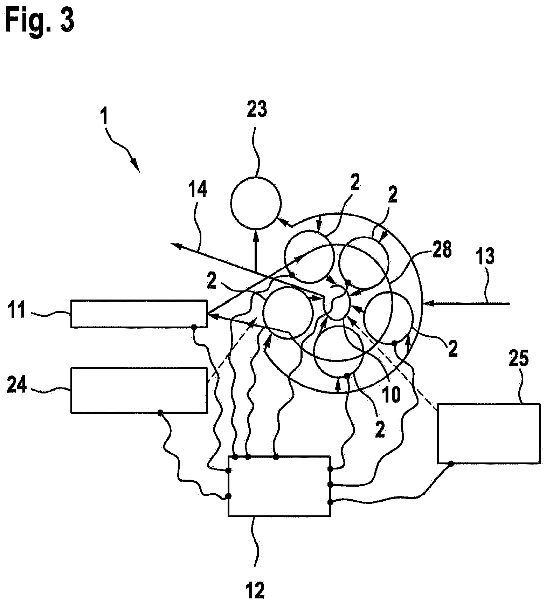

Figur 1 zeigt eine Granuliereinheit 2 einer Wirbelschichtanlage 1 (vergleiche Figuren 2 und 3) gemäß einem Ausführungsbeispiel der Erfindung. Der Granuliereinheit 2 wird ein Fluid, insbesondere ein Gas, besonders bevorzugt Luft, über eine nicht gezeigte Luftaufbereitungsanlage mit Wärmeübertrager, Filter und Fremdluftzufuhr zugeführt. Die Gaszuführung in die Granuliereinheit 2 erfolgt über eine Fluidzufuhr 6, wobei die Fluidzufuhr 6 in einem Boden 15 der Granuliereinheit 2 angeordnet ist. Über dem Boden 15 ist insbesondere ein scheibenförmiger Gasverteilerboden (30) angeordnet, der die zugeführte Luft großflächig über den gesamten Querschnitt des Wirbelschichtbehälters 3 verteilt.

Der Boden 15 ist insbesondere kreisförmig ausgebildet und grenzt einen hohlzylinderförmigen Wirbelschichtbehälter 3 ab. Außerdem ist der Wirbelschichtbehälter 3 durch einen ebenfalls kreisförmigen Deckel 16 abgegrenzt. Somit erstrecken sich Seitenwände 17 des Wirbelschichtbehälters 3 zwischen dem Boden 15 und dem Deckel 16. Der Boden 15 und der Deckel 16 sind insbesondere parallel zueinander angeordnet.

Parallel unterhalb des Deckels 16 ist ein scheibenförmiger Filterboden 29 vorgesehen. Dieser Filterboden 29 umfasst und trägt mindestens einen Filter 27, der an einem Fluidauslass 7 angeordnet ist. Das über den Fluideinlass 6 im Boden 15 eingebrachte Gas ist somit über den Filter 27 und über den Fluidauslass 7 aus der Granuliereinheit 2 entnehmbar. Auf diese Weise ist ein Fluidstrom, insbesondere ein Gasstrom, innerhalb des Wirbelschichtbehälters 3 generierbar. Weiterhin weist die Granuliereinheit 2 Einspritzdüsen 8 auf, die insbesondere ins Innere des Wirbelschichtbehälters 3 ragen. In Figur 1 sind die Einspritzdüsen als seitliche Einspritzdüsen (Side Spray) ausgebildet. Alternativ oder zusätzlich können Einspritzdüsen 8 innerhalb des Bodens 15 (Bottom Spray) und/oder innerhalb des Deckels 16 (Top Spray) angeordnet sein. Die Einspritzdüsen liefern einen Bearbeitungsstoff, insbesondere eine Granulierflüssigkeit, der für den Prozess innerhalb des Wirbelschichtbehälters 3 verwendet werden soll. Der von dem Wirbelschichtbehälter 3 umschlossene Raum zwischen dem Boden 15 und dem Deckel 16, insbesondere zwischen dem Gasverteilerboden 30 und dem Filter 27, bildet den Prozessraum, der somit von dem über den Fluideinlass 6 eingegebenen Gas durchströmt wird. Der Prozessraum erstreckt sich von einer Mittelachse des Wirbelschichtbehälters 3 zur Seitenwand 17 und ist rotationssymmetrisch, besonders vorteilhaft zylinderförmig, ausgeführt. In den Prozessraum ist über einen Einlass 4, der durch die Seitenwand 17 reicht, ein pulverförmiger Feststoff eingebbar. Über einen Auslass 5, der ebenfalls durch die Seitenwand 17 reicht, ist ein fertiges Produkt, insbesondere ein trockenes Granulat, aus dem Wirbelschichtbehälter 3 entnehmbar. Der pulverförmige Feststoff und das Granulat werden vorzugsweise pneumatisch in und aus der Granuliereinheit durch Saugförderung, oder alternativ gravimetrisch mit geeigneten Einrichtungen, befördert.

Das durch den Prozessraum hindurchströmende Gas, das den Prozessraum von dem in dem Boden 15 vorhandenen Fluideinlass 6 zu dem in dem Deckel 16 vorhandenen Fluidauslass 7 durchströmt, versetzt den über den Einlass 4 in den Prozessraum eingegebenen pulverförmigen Feststoff in einem fluidähnlichen Bewegungszustand. Dabei entsteht ein intensiver Wärmeaustausch sowie Stoffaustausch, welche durch einen sehr intensiven Mischprozess ermöglicht werden. Dieser Mischprozess ermöglicht es auch, das im Prozessraum befindliche fluidisierte Pulver mit einer über die Einspritzdüsen 8 in den Prozessraum eingesprühten Granulierflüssigkeit zu vermischen. Das Mischen von fluidisiertem Pulver mit der eingesprühten Granulierflüssigkeit ermöglicht das Agglomerieren mehrerer Einzelpartikel des Pulvers zu Partikelkollektiven (Agglomeraten). Durch den gleichzeitigen intensiven Wärmetransport verdunstet ein Großteil der Granulierflüssigkeit an der Agglomeratoberfläche und teilweise in den Poren der Agglomerate, sodass diese trocknen. Dabei bleibt jedoch etwas Flüssigkeit in den Agglomeraten zurück und ermöglicht so die Haftung der Einzelpartikel untereinander. Das somit hergestellte Kollektiv von Agglomeraten wird als Granulat bezeichnet.

Die Granulierflüssigkeit kann, wie zuvor beschrieben, auf unterschiedliche Weisen dem Prozessraum zugeführt werden. So können insbesondere die Verfahren Top Spray, Bottom Spray und Side Spray verwendet werden. Bei den Einspritzdüsen 8 handelt es sich vorteilhafterweise um Dreistoffdüsen.

Der Prozess der Wirbelschichtgranulierung innerhalb jeder Granuliereinheit 2 wird mit nicht dargestellten Messeinrichtungen in den Leitungen der Zuluft und der Abluft, die den Fluideinlass 6 mit einer Luftversorgung und den Fluidauslass 7 mit einer Luftentsorgung verbinden, über und unter dem Boden 15, im Prozessraum, sowie vor und nach dem Filter 27 kontrolliert. Dabei bestimmen die Messeinrichtungen vorteilhafterweise Daten, die Aussagen über den Gasmassenstrom zwischen Fluideinlass 6 und Fluidauslass 7, über eine Beladung des Gases mit Feuchtigkeit, über eine Temperatur des Gases und des Feststoffes, über einen Druckverlust innerhalb des Bodens 15, der bevorzugt als Luftverteilerboden ausgebildet ist, sowie an dem Filter 27, und optional über die Partikelgrößenverteilung sowie über die Produktfeuchte erlauben. Diese Daten werden an eine Steuereinheit 12 (vgl. Fig. 3) der Wirbelschichtanlage 1 übertragen. Dabei ist vorgesehen, dass eine Steuereinheit 12 übergeordnet für sämtliche vorhandenen Granuliereinheiten 2 verwendet wird. Über die Steuereinheit 12 sind entsprechende Stellglieder der einzelnen Granuliereinheiten 2 beeinflussbar, um den Prozess in den einzelnen Granuliereinheiten 2 innerhalb von vorgegebenen Prozessgrenzen stabil und zuverlässig zu betreiben. Dazu lassen sich Ventile zum Steuern eines Transports von Material durch den Einlass 4 sowie den Auslass 5, zum Steuern des Fluidflusses, insbesondere der Gasströmung, zwischen Fluideinlass 6 und Fluidauslass 7, und zum Steuern eines Reinigungssystems, passend einstellen. Die Funktionalität des Reinigungssystems, insbesondere eines Reinigungsmoduls 11, wird nachfolgend mit Bezug auf Figur 2 beschrieben.

Figur 2 zeigt eine alternative Ausführung der Granuliereinheiten 2 der Wirbelschichtanlage 1. Die alternative Ausführung ist mit der in Figur 1 gezeigten Ausführung identisch, wobei lediglich ein Unterschied hinsichtlich des Einlasses 4 und dem Auslasses 5 zwischen Figur 1 und Figur 2 besteht. In Figur 1 ist in der Seitenfläche 3 jeweils eine eigene Öffnung für den Einlass 4 und den Auslass 5 vorhanden. In der in Figur 2 gezeigten Alternative ist eine einzige Öffnung innerhalb der Seitenfläche 3 vorhanden, wobei der Einlass 4 und der Auslass 5 über diese eine gemeinsame Öffnung in der Seitenfläche 3 realisiert sind.

Figur 3 zeigt eine erste Alternative einer Wirbelschichtanlage 1 gemäß dem Ausführungsbeispiel der Erfindung. Die gezeigte Wirbelschichtanlage 1 weist fünf Granuliereinheiten 2 auf. Die Wirbelschichtanlage 1 weist insbesondere zumindest zwei, besonders vorteilhaft bis zu zehn oder mehr einzelne Granuliereinheiten 2 auf. Sämtliche Granuliereinheiten 2 lassen sich parallel betreiben. Dabei ist bevorzugt vorgesehen, dass mindestens eine Granuliereinheit bis zu ihrer Aktivierung in einem Stand-by-Modus verbleibt. Weiterhin ist vorgesehen, dass die Granulationsanlage 1 eine Einrichtung zur Entsorgung von Ausschussmaterial aufweist. Eine solche Abfallsammelvorrichtung 23 dient vorteilhafterweise zum Sammeln von sowohl Ausschuss innerhalb des in die Granuliereinheiten 2 einzugebenden pulverförmigen Feststoffs sowie innerhalb des fertigen Granulats.

Die einzelnen Granuliereinheiten 2 weisen bevorzugt eine gemeinsame Luftversorgung 24 auf. Die Luftversorgung 24 ist mit allen Fluideinlässen 6 der Granuliereinheiten 2 verbunden. Es ist jedoch bevorzugt vorgesehen, dass über die Steuereinheit 12 für jede Granuliereinheit 2 eine Menge und/oder eine Feuchte und/oder eine Eingangstemperatur des durch den Fluideinlass 6 strömenden Gases individuell eingestellt werden kann. Alternativ kann jede Granuliereinheit 2 eine eigene Luftversorgung 24 aufweisen.

Die Wirbelschichtanlage 1 weist außerdem ein Reinigungsmodul 11 auf. Das Reinigungsmodul erlaubt insbesondere eine Reinigung ohne manuelle Nacharbeitung (Clean in Place, CIP) oder alternativ eine Reinigung mit manueller Nacharbeitung (Wipe in Place, WIP). Das Reinigungsmodul ist über eine Reinigungsleitung 28 mit jeder Granuliereinheit 2 zur vollautomatischen Reinigung verbunden. Die Verbindung ist dazu vorteilhafterweise dauerhaft, kann alternativ aber auch lediglich zur Reinigung hergestellt werden. Die Reinigungsleitung 28 ist bevorzugt über Ventile mit jeder einzelnen Granuliereinheit 2 verbunden und versorgt bei Bedarf und selektiv ansteuerbar idealerweise eine oder mehrere Reinigungsdüsen mit einem Reinigungsmedium, die vorteilhafterweise in der Seitenwand 17 und/oder im Fluideinlass 6 und/oder im Fluidauslass 7 und/oder im Deckel 16 und/oder im Filterboden 29 und / oder im Boden 15 und/oder im Einlass 4 und/oder im Auslass 5 und/oder in der Zuführleitung 9 und/oder in der Auslassleitung 10 und/oder im Moduleinlass 13 und/oder im Modulauslass 14 und/oder im Gesamteinlass 19 angeordnet sind, wobei diese Ventile von der Steuereinheit 12 ansteuerbar sind. Somit lässt sich von der Steuereinheit 12 jede Granuliereinheit 2 einzeln reinigen, wobei ein Betrieb der verbleibenden Granuliereinheiten 2 nicht beeinträchtigt ist.

Die Wirbelschichtanlage 1 umfasst weiterhin eine Leitung 9, welche ringsegmentförmig oder ringförmig ausgebildet ist. Die Einlassleitung 9 umschließt die ringförmig angeordneten Granuliereinheiten 2 und verbindet sämtliche Einlässe der Granuliereinheiten 2. Dabei ist vorgesehen, dass jeder Einlass 4 einer Granuliereinheit 2 über ein von der Steuereinheit 12 ansteuerbares Ventil mit der Einlassleitung 9 verbunden ist. Somit ist über die Steuereinheit 12 steuerbar, welche Granuliereinheit 2 mit einem pulverförmigen Feststoff versorgt werden soll. Die Einlassleitung 9 ist außerdem mit der Abfallsammelvorrichtung 23 verbunden, um nicht geeignetes Material an die Abfallsammelvorrichtung 23 zu übergeben. Auch hier ist vorgesehen, dass ein Ventil zwischen Abfallsammelvorrichtung 23 und Einlassleitung 9 vorhanden ist, wobei dieses Ventil von der Steuervorrichtung 12 steuerbar ist. Schließlich ist vorgesehen, dass die Einlassleitung 9 mit einem Moduleinlass 13 verbunden ist. Über den Moduleinlass 13 ist die Einlassleitung 9 mit einem Material, insbesondere mit einem pulverförmigen Feststoff, befüllbar.

Die Wirbelschichtanlage 1 weist außerdem eine Auslassleitung 10 auf. Die Auslassleitung 10 ist ringsegmentförmig ausgebildet und wird von den ringförmig angeordneten Granuliereinheiten 2 umschlossen. Dabei ist vorgesehen, dass die Auslassleitung 10 die Auslässe 5 aller Granuliereinheiten 2 verbindet. Diese Verbindung erfolgt über jeweils ein eigenes Ventil, wobei jedes Ventil unabhängig von jedem anderen Ventil von der Steuervorrichtung 12 ansteuerbar ist. Die Auslassleitung 10 ist außerdem mit der Abfallsammelvorrichtung 23 verbunden, um Ausschuss innerhalb des in den Granuliereinheiten 2 hergestellten Granulats zu entsorgen. Auch hier ist bevorzugt ein Ventil vorhanden, das von der Steuereinheit 12 steuerbar ist. Schließlich ist die Auslassleitung 10 mit einem Modulauslass 14 verbunden. Hier ist wiederum bevorzugt ein Ventil vorhanden, wobei das Ventil von der Steuereinheit 12 steuerbar ist.

Die Wirbelschichtanlage 1 weist mit der Steuereinheit 12 eine übergeordnete Gesamtregelungseinrichtung auf, die neben der Vielzahl von Granuliereinheiten 2 insbesondere auch das Vorbereitungsmodul 13 und das Reinigungsmodul 11 ansteuert.

Bevorzugt ist außerdem eine Transportluftversorgung 25 vorhanden. Die Transportluftversorgung 25 erlaubt ein pneumatisches Entfernen des Granulats aus den Granuliereinheiten 2, indem das Granulat in die Auslassleitung 10 gesaugt wird. Hierzu dient die Transportluftversorgung 25. Über die Transportluftversorgung 25 ist außerdem sichergestellt, dass das Granulat zu dem Modulauslass 14 transportiert werden kann.

Nachfolgend wird beispielsweise beschrieben, wie die Wirbelschichtanlage 1 verwendet werden kann:
Die Granuliereinheiten 2 werden seriell, nacheinander in einem Abstand von mindestens zehn bis 600 Sekunden oder mehr mit pulverförmigem Feststoff, pneumatisch mit der Luftversorgung 24 befüllt. Hierbei erlaubt der Luftstrom innerhalb jeder Granuliereinheit 2 einen Sog zu erzeugen, über den der pulverförmige Feststoff in den Wirbelschichtbehälter 3 saugbar ist. Alternativ lassen sich die Granuliereinheiten 2 gravimetrisch befüllen. Sobald der Befüllvorgang einer einzelnen Granuliereinheit 2 abgeschlossen ist, startet die Wirbelschichtgranulation in dieser Granuliereinheit 2. Die Entleerung des Granulats aus den Granuliereinheiten 2 erfolgt ebenfalls pneumatisch mit der Transportluftversorgung 25, indem die Granuliereinheit keinen Sog mehr erzeugt, die Transportluftversorgung 25 jedoch einen Sog in der Auslassleitung 10. Alternativ kann das Entleeren auch gravimetrisch erfolgen. Das Entleeren erfolgt seriell im selben Zeitabstand wie die Befüllung, wobei die Reihenfolge und der Zeitabstand der Entleerung der einzelnen Granuliereinheiten 2 entsprechend der Reihenfolge der Befüllung ist. Jede Granuliereinheit 2 wird bevorzugt unmittelbar nach der Entleerung wieder mit pulverförmigem Feststoff befüllt. Der Zeitpunkt für die Entleerung kann alternativ durch erreichen eines Abbruchkriteriums (Temperaturen oder Feuchtigkeit im Prozessraum) des Granulier- und Trocknungsprozesses bestimmt werden.

Nachdem eine Granuliereinheit 2 einmal befüllt wurde und wieder entleert wurde, ist ein Zyklus dieser Granuliereinheit 2 durchfahren worden. Jede Granuliereinheit 2 kann Materialabhängig über mehrere Zyklen ohne Reinigung betrieben werden. Nach dem Erreichen einer vordefinierten maximalen Zykluszahl initiiert die Steuervorrichtung 12 eine vollautomatische Reinigung oder eine Vorreinigung der jeweiligen Granuliereinheit 2 mittels des Reinigungsmoduls 11. Nach Abschluss der Reinigung oder der Vorreinigung kann die Granuliereinheit 2 direkt wieder betrieben oder falls nötig manuell endgereinigt werden. Der Reinigungsprozess mittels des Reinigungsmoduls 11 läuft in einer Weise ab, dass währenddessen die übrigen Granuliereinheiten 2 weiter betrieben werden können.

Die übergeordnete Gesamtregelungseinrichtung in Form der Steuereinheit 12 steuert die Luftversorgung 24 für das Prozessgas, das bedeutet, das Fluid, das über die Fluidzufuhr 6 jeder Granuliereinheit 2 zugeführt wird, sowie die Transportluftversorgung 25. Weiterhin steuert die Gesamtregelungseinrichtung in Form der Steuereinheit 12 sämtliche Ventile für den Produkttransport und/oder Materialtransport, welche mit der Einlassleitung 9 und/oder der Auslassleitung 10 verbunden sind. Schließlich steuert die Gesamtregelungseinrichtung in Form der Steuereinheit 12 das Reinigungsmodul 11 sowie sämtliche Stellglieder, die den Prozess der Wirbelschichtgranulation in den einzelnen Granuliereinheiten 2 beeinflussen. Auf diese Weise ist ermöglicht, dass die Prozessparameter in vorgegebenen Prozessgrenzen stabil und zuverlässig gehalten werden, wodurch die Wirbelschichtgranulation sehr sicher und zuverlässig erfolgt. Weiterhin überwacht die Gesamtregelungseinrichtung in Form der Steuereinheit 12 die Wirbelschichtanlage 1 in der Form, dass alle Messdaten der in den Granuliereinheiten 2 befindlichen Sensoren aufgezeichnet und ausgewertet werden. Außerdem können die Messdaten auf einer Mensch-Maschine-Schnittstelle für einen Anwender dargestellt werden. Auf diese Weise lassen sich potenziell auftretende technische Probleme, welche potenziell zu technischen Störungen oder Qualitätsschwankungen des Prozess führen können, rasch erkennen. Insbesondere sei hier der zeitliche Verlauf des Druckverlustes über dem Gasverteilerboden 30 jeder Granuliereinheit 2 oder über dem Filter 27 jeder Granuliereinheit 2 zu erwähnen. Wird der zeitliche Anstieg dieses Parameters registriert und als problematisch erkannt, so kann die Steuereinheit 12 selbstständig entscheiden, welche Granuliereinheit 2 als nächstes automatisch gereinigt werden soll und/oder eine Meldung an den Benutzer ausgeben, dass eine entsprechende Reinigung durchzuführen ist. Auch die Überwachung der Sprührate der Granulierflüssigkeit mittels der Einspritzdüsen 8 kann als Indikator für den Zustand der Einspritzdüsen 8 oder der dahinterstehenden Leitungen verwendet werden. Die Granulationsanlage 1 ist somit in der Lage, ihren technischen Zustand selbstständig zu überwachen und zu optimieren, wodurch Ausfallzeiten minimiert sind.

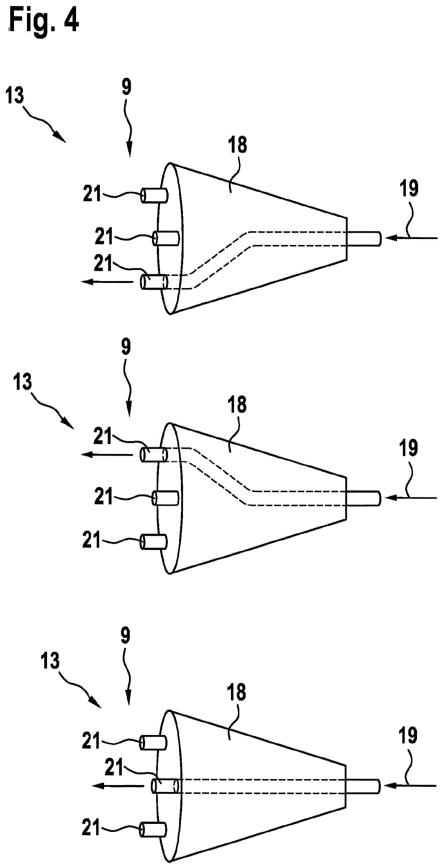

Figur 4 zeigt schematisch eine erste Alternative eines Moduleinlasses 13. Dabei ist der Moduleinlass 13 als Weiche 18 ausgebildet. Die Weiche 18 hat in dem in Figur 4 gezeigten Beispiel eine Vielzahl von Einzelausgängen 21, die zusammen die Einlassleitung 9 ergeben. Dabei ist jeder Einzelausgang 21 separat mit einem Gesamteinlass 19 verbindbar. In Figur 4 sind dazu 3 verschiedene Wege gezeigt, jeder einzelne Einzelausgang 21 mit dem Gesamteinlass 19 verbunden werden kann. Die Einlassleitung 9 umfasst somit eine Vielzahl von einzelnen Leitungen, die jeden einzelnen Einzelausgang 21 mit einer Granuliereinheiten 2 verbindet.

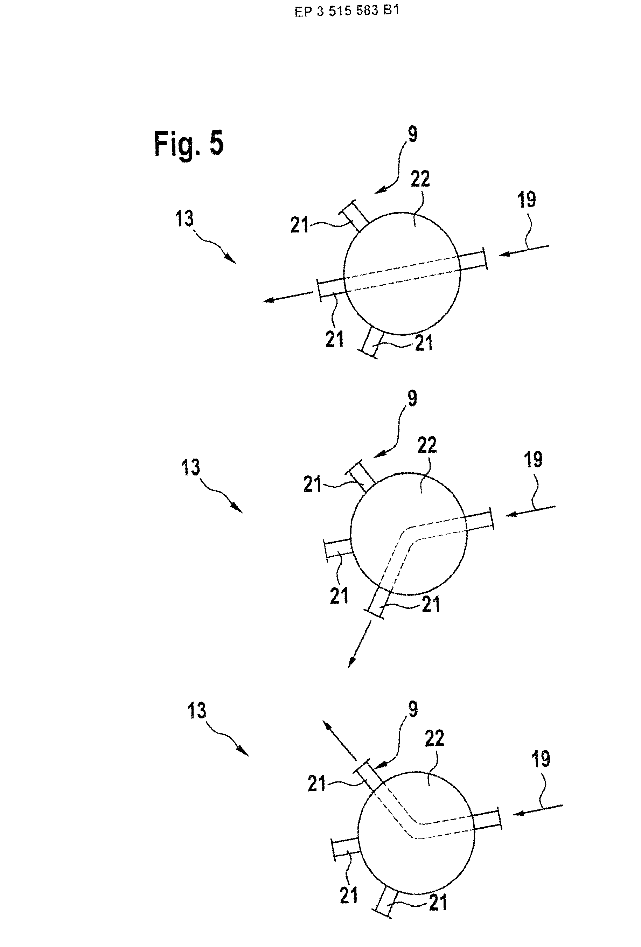

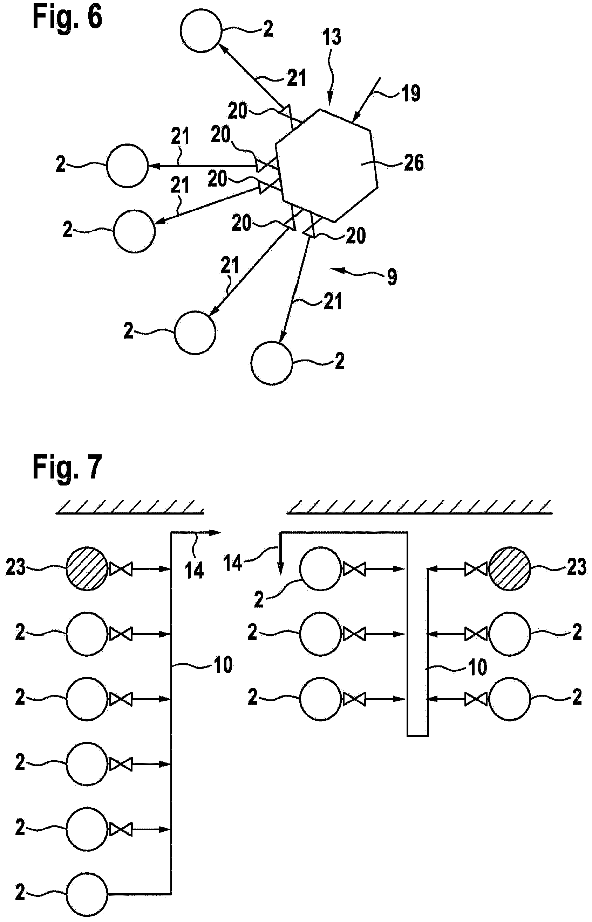

Figur 5 zeigt schematisch eine zweite Alternative des Moduleinlasses 13. Dabei ist der Moduleinlass 13 als Umschalteinheit 22 ausgebildet. Wie bereits in dem in Figur 4 gezeigten Beispiel ist wiederum eine Vielzahl von Einzelausgängen 21 vorhanden, wobei die Einzelausgang 21 gemeinsam die Einlassleitung 9 bilden. Somit ist wiederum bevorzugt vorgesehen, dass jeder Einzelausgang 21 separat mit jeweils einer Granuliereinheiten 2 verbunden ist. Durch die Umschalteinheit 22 ist jeder Einzelausgang 21 einzeln mit dem Gesamteinlass 19 verbindbar. Figur 6 zeigt schematisch eine dritte Alternative des Moduleinlasses 13. Hierbei umfasst der Moduleinlass 13 einen Zentralverteiler 26, der eine Vielzahl von Einzelventilen 20 aufweist. Über den Zentralverteiler 26 ist jedes Einzelventil 20 mit dem Gesamteinlass 19 verbunden. Jedes Einzelventil 20 steuert einen Einzelausgang 21, wobei wiederum jeder Einzelausgang 21 mit genau einer Granuliereinheit 2 verbunden ist. Somit kann durch Ansteuerung jedes Einzelventils 20 die zugeordnete Granuliereinheiten 2 mit dem Gesamteinlass 19 verbunden werden.

Figur 7 zeigt schematisch zwei bevorzugte Alternativen eines Modulauslasses 14 der Wirbelschichtanlagen 1. So ist einerseits ermöglicht, eine linear verlaufende Ausgangsleitung 10 zu verwenden, die mit dem Modulauslass 14 verbunden ist. Dies ist insbesondere dann vorteilhaft, wenn die Granuliereinheiten 2 und insbesondere auch eine eventuell vorhandene Abfallsammelvorrichtung 23 seriell, das bedeutet insbesondere in einer Reihe, angeordnet sind. Alternativ kann die Auslassleitung 10 wie in Figur 7 gezeigt U-förmige verlaufen, wenn die Granuliereinheiten 2 und insbesondere auch eine eventuell vorhandene Abfallsammelvorrichtung 23 entlang von zwei oder mehreren Reihen angeordnet sind. Der Modulauslass ist wiederum bevorzugt mit der Auslassleitung 10 verbunden.

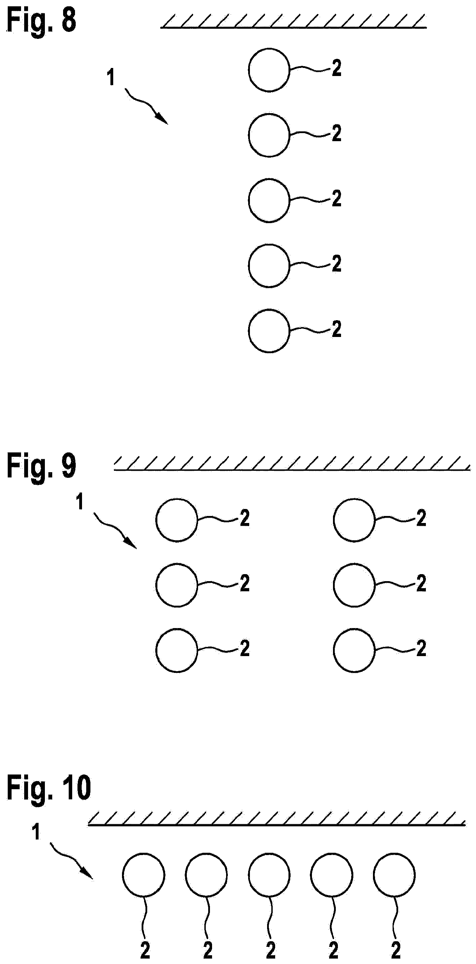

Die Figuren 8 bis 10 zeigen bevorzugt alternative Anordnungsbeispiele zu der in Figur 3 gezeigten Ringanordnung der Granuliereinheiten 2. So sind in den Figuren 8 bis 10 Wirbelschichtanlagen 1 lediglich durch die Granuliereinheiten 2 repräsentiert. Die übrigen in Figur 3 gezeigten Komponenten werden der besseren Übersichtlichkeit halber in Figuren 8 bis 10 nicht gezeigt.

So zeigt Figur 8 eine schematische Anordnung der Wirbelschichtanlage 1 der Art, dass die Granuliereinheiten 2 spaltenförmig angeordnet sind. Dies weist insbesondere den Vorteil auf, dass jede Granuliereinheit 2 von außen einfach zugänglich ist, so dass diese zur Wartung und/oder manuellen Reinigung leicht von einem Benutzer erreichbar ist. In Figur 9 bilden die Granuliereinheiten 2 zwei parallel verlaufende Spalten. Figur 10 zeigt schließlich, dass die Granuliereinheiten 2 reihenförmig angeordnet sind. Auch hier ergeben sich die zuvor beschriebenen Vorteile hinsichtlich der Erreichbarkeit sowie der vereinfachten Wartung und/oder manuellen Reinigung. Ebenso ist möglich, mehrere Granuliereinheiten 2 reihenförmig parallel anzuordnen.

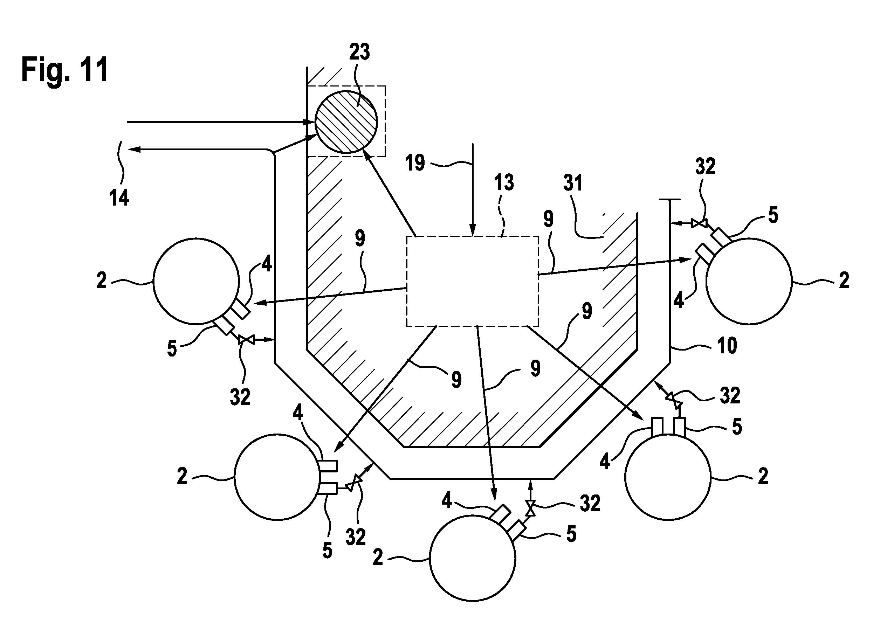

Die Figur 11 zeigt eine weitere Alternative einer Wirbelschichtanlage 1 gemäß dem Ausführungsbeispiel der Erfindung. Die Granuliereinheiten 2 sind halbkreisförmig an einer polygonförmigen, halbkreisnachempfundener Technikwand 31 angeordnet. Der Einlass 4 und der Auslass 5 sind zur Technikwand 31 hin ausgerichtet. Der Einlass 4 für Pulver einer jeden Granuliereinheit 2 ist mit einer eigenen Einlassleitung 9 durch die Technikwand 31 hindurch verbunden. Jede Einlassleitung 9 ist mit dem Moduleinlass 13 verbunden. Der Gesamteinlass 19 für Pulver ist mit dem Moduleinlass 13 verbunden. Der Auslass 5 einer jeden Granuliereinheit ist mit der Auslassleitung 10 verbunden, wobei hier in vorteilhafterweise ein Absperrventil 32 zwischengeschaltet ist, um Wartungsarbeiten am Auslass 5 der Granuliereinheit 2 bei Bedarf durchzuführen, während die andere Granuliereinheiten 2 in Betrieb sind und ggf. Produkt durch die Auslassleitung 10 transportiert wird. Die Auslassleitung 10 transportiert das Produkt zum Modulauslass 14. Die Abfallsammelvorrichtung 23 ist sowohl über die Einlassleitung 9 mit dem Moduleinlass 13, sowie alternativ mit der Auslassleitung 10, idealerweise mit dem Modulauslass 14 verbunden. Steuerungseinheit 12, Luftversorgungen 24 und 25 und Reinigungssystem 11 sind der besseren Übersicht halber nicht gezeigt.

Die Wirbelschichtanlage 1 weist die folgenden Vorteile auf:
- kein Scale up von Laborversuchen zu Produktionsprozessen, da die Anlage für beide Zwecke verwendet werden kann,
- nur ein Einlass für eine Vielzahl von pharmazeutischen Pulver zu dem Moduleinlass 13,
- 100 Prozent Verfolgungsmöglichkeit (Traceability) jeder Batch,
- keine Querkontamination zwischen den einzelnen Batches, da die Granuliereinheiten 2 komplett voneinander getrennt und unabhängig betrieben werden,
- einzelne Granuliereinheiten 2 während des Betriebs der übrigen Granuliereinheiten 2 sind automatisch reinigbar, ohne dass die Wirbelschichtanlage 1 geöffnet werden muss; alternativ sind die Granuliereinheiten 2 vorreinigbar, sodass eine manuelle Nachreinigung erfolgen muss; in jedem Fall erfolgt das Reinigung und/oder Vorreinigen im laufenden Betrieb der übrigen Granuliereinheiten 2,
- die Fluidversorgung insbesondere Luftversorgung jeder Granuliereinheit 2 ist einzeln und unabhängig regelbar; es lassen sich für jede Granuliereinheit 2 eigene Parameter, wie insbesondere Luftmenge, Luftfeuchtigkeit und Lufttemperatur einstellen,
- Versorgung der einzelnen Granuliereinheiten 2, sowie der Ausschussbehälter, bevorzugt über einzelne Ringleitungen, wobei die Ringleitungen ringförmig oder ringsegmentförmig ausgestaltet sein können,
- Möglichkeit der Ausschleusung vor und nach dem Wirbelschichtgranulieren,
- die beschriebene Einlassleitung 9 und die Auslassleitung 10 verringert die Zahl benötigter Ventile und erhöht die Prozessflexibilität,
- verschiedene Möglichkeiten Bearbeitungsstoffe, insbesondere Granulierflüssigkeiten, einzusprühen: Top Spray, Bottom Spray, Side Spray,
- Einbindung in ein Steuerungskonzept und/oder Regelungskonzept der Granuliereinheiten 2, was bedeutet
   - übergeordnete Regelungseinrichtung zur Integration der einzelnen Granuliereinheiten 2 in die Wirbelschichtanlage 1,
   - Realisierung von Closed-Loop-Control mit übergeordneter Regelungseinrichtung für die einzelnen Granuliereinheiten 2.
- Ein effizientes, abgestimmtes Prozessanalysetechnikkonzept (PAT-Konzept) in allen Granuliereinheiten 2
   - Continous Process Verification (CVP)
   - Soft Sensor Modelling
   - PAT (Process Analytical Technology)
   - SPC (Statistic Process Control) mittels MVDA (Multi variate Data Analysis) und univariater Datenauswertung
   - Datenbanksystem zur Datenverwaltung, Sammlung von Daten, und Auswertung von Daten
- automatische Erkennung von möglichen auftretenden technischen Problemen der einzelnen Granuliereinheiten 2 und Meldung an einen Benutzer, Darstellung von Empfehlungen und vorteilhafterweise selbstständiges Ergreifen von Maßnahmen, insbesondere Reinigung, zur Vermeidung schwerwiegenderer Probleme,
- Minimierung von Ausfallzeiten und Menge an Ausschussmaterial,
- Entwicklung und Produktion auf einer Anlage,
- automatisches Durchführen von Versuchsprogrammen (DoE, etc.) über einen kompletten Wirbelschicht-Kalkulation-Prozesses nach Vorgaben eines Benutzers
   - verschiedene Formulierungen
   - verschiedene Füllmengen
   - verschiedene Betriebsparameter

## Patentansprüche

1. Wirbelschichtanlage (1) umfassend
- eine Vielzahl von funktional parallel angeordneten Granuliereinheiten (2) für die Herstellung von pharmazeutischen Granulaten, wobei jede Granuliereinheit (2) aufweist:
- einen Wirbelschichtbehälter (3) mit einem Deckel (16), einem Boden (15) und einer zwischen dem Deckel (16) und dem Boden (15) erstreckten Seitenwand,
- einen Einlass (4) in der Seitenwand des Wirbelschichtbehälters (3) zum Einbringen von pulverförmigem Feststoff sowie einen
- Auslass (5) in der Seitenwand des Wirbelschichtbehälters (3) zur Entnahme von Pulver oder eines fertigen Produkts,
- eine Fluidzufuhr (6) in dem Boden (15) sowie eine Fluidabfuhr (7) in dem Deckel (16) des Wirbelschichtbehälters (3), und
- zumindest eine Einspritzdüse (8) zum Einspritzen eines Bearbeitungsstoffs in den Wirbelschichtbehälter (3), und
- eine Steuereinheit (12) zum Einstellen von Prozessbedingungen innerhalb jeder Granuliereinheit (2).

2. Wirbelschichtanlage (1) nach Anspruch 1, **gekennzeichnet durch** eine Einlassleitung (9), über die die Einlässe (4) aller Granuliereinheiten (2) verbunden sind.

3. Wirbelschichtanlage (1) nach einem der vorhergehenden Ansprüche, **gekennzeichnet durch** eine Auslassleitung (10) über die die Auslässe (5) aller Granuliereinheiten (2) verbunden sind.

4. Wirbelschichtanlage (1) nach einem der vorhergehenden Ansprüche, **gekennzeichnet durch** ein Reinigungsmodul (11), das mit jeder Granuliereinheit (2) wirkverbunden ist und mit dem jede Granuliereinheit (2) unabhängig von jeder anderen Granuliereinheit (2) reinigbar ist.

5. Wirbelschichtanlage (1) nach Anspruch 4, **dadurch gekennzeichnet, dass** das Reinigungsmodul (11) zum Durchführen eines Reinigens mit manuellem Nacharbeiten oder eines Reinigens ohne manuellem Nacharbeiten ausgebildet ist.

6. Wirbelschichtanlage (1) nach einem der vorhergehenden Ansprüche, **gekennzeichnet durch** zumindest einen Moduleinlass (13), wobei der Moduleinlass (13) mit den Einlässen (4) aller Granuliereinheiten (2) verbunden ist, und wobei der Moduleinlass (13) von der Steuereinheit (12) ansteuerbar ist.

7. Wirbelschichtanlage (1) nach Anspruch 6, **dadurch gekennzeichnet, dass** der Moduleinlass (13) als zentrales Verteilungsmodul (18) und/oder Puffermodul und/oder Umschaltventil und/oder Weiche und/oder Ringleitung ausgebildet ist.

8. Wirbelschichtanlage (1) nach einem der vorhergehenden Ansprüche, **gekennzeichnet durch** zumindest einen Modulauslass (14), wobei der Modulauslass (14) mit den Auslässen (5) aller Granuliereinheiten (2) verbunden ist, und wobei der Modulauslass (14) von der Steuereinheit (12) ansteuerbar ist.

9. Wirbelschichtanlage (1) nach einem der vorhergehenden Ansprüche, **dadurch gekennzeichnet, dass** die zumindest eine Einspritzdüse (8) in dem Boden (15) und/oder in dem Deckel (16) und/oder in der Seitenwand (17) der Granuliereinheit (2) angeordnet ist.

10. Verfahren zur semi-kontinuierlichen Herstellung von pharmazeutischen Granulaten in einer Wirbelschichtanlage (1) nach Anspruch 1, **dadurch gekennzeichnet, dass** Granuliereinheiten (2) der Wirbelschichtanlage (1) unabhängig voneinander betrieben und/oder gereinigt werden.

## Claims

1. Fluidized bed system (1) comprising
- a plurality of granulation units (2) arranged functionally in parallel for producing pharmaceutical granules, wherein each granulation unit (2) has:
- a fluidized bed container (3) with a lid (16), a bottom (15) and a side wall extending between the lid (16) and the bottom (15),
- an inlet (4) in the side wall of the fluidized bed container (3) for introducing powdered solid material and an
- outlet (5) in the side wall of the fluidized bed container (3) for the removal of powder or a finished product,
- a fluid supply (6) in the bottom (15) and a fluid discharge (7) in the lid (16) of the fluidized bed container (3), and
- at least one injection nozzle (8) for injecting a processing material into the fluidized bed container (3), and
- a control unit (12) for adjusting process conditions inside each granulation unit (2).

2. Fluidized bed system (1) according to claim 1,
**characterized by** an inlet line (9) via which the inlets (4) of all of the granulation units (2) are connected.

3. Fluidized bed system (1) according to either of the preceding claims, **characterized by** an outlet line (10) via which the outlets (5) of all of the granulation units (2) are connected.

4. Fluidized bed system (1) according to any of the preceding claims, **characterized by** a cleaning module (11) which is operatively connected to each granulation unit (2) and by means of which each granulation unit (2) can be cleaned independently of every other granulation unit (2).

5. Fluidized bed system (1) according to claim 4,
**characterized in that** the cleaning module (11) is designed to carry out cleaning with manual reworking or cleaning without manual reworking.

6. Fluidized bed system (1) according to any of the preceding claims, **characterized by** at least one module inlet (13), the module inlet (13) being connected to the inlets (4) of all of the granulation units (2), and it being possible to control the module inlet (13) by means of the control unit (12).

7. Fluidized bed system (1) according to claim 6,
**characterized in that** the module inlet (13) is designed as a central distribution module (18) and/or a buffer module and/or a change-over valve and/or a switch and/or a loop line.

8. Fluidized bed system (1) according to any of the preceding claims, **characterized by** at least one module outlet (14), the module outlet (14) being connected to the outlets (5) of all of the granulation units (2), and it being possible to control the module outlet (14) by means of the control unit (12).

9. Fluidized bed system (1) according to any of the preceding claims, **characterized in that** the at least one injection nozzle (8) is arranged in the bottom (15) and/or in the lid (16) and/or in the side wall (17) of the granulation unit (2).

10. Method for semi-continuously producing pharmaceutical granules in a fluidized bed system (1) according to claim 1, **characterized in that** granulation units (2) of the fluidized bed system (1) are operated and/or cleaned independently of one another.

## Revendications

1. Installation de lit fluidisé (1), comprenant
- une pluralité d'unités de granulation (2) disposées fonctionnellement en parallèle pour la production de granulés pharmaceutiques, dans laquelle chaque unité de granulation (2) présente :
- un récipient de lit fluidisé (3) comportant un élément de recouvrement (16), un fond (15) et une paroi latérale s'étendant entre l'élément de recouvrement (16) et le fond (15),
- une entrée (4) dans la paroi latérale du récipient de lit fluidisé (3) pour l'introduction de matières solides pulvérulentes, ainsi qu'une
- sortie (5) dans la paroi latérale du récipient de lit fluidisé (3) pour le prélèvement de poudre ou d'un produit fini,
- une amenée de fluide (6) dans le fond (15) ainsi qu'une évacuation de fluide (7) dans l'élément de recouvrement (16) du récipient de lit fluidisé (3), et
- au moins une buse d'injection (8) pour l'injection d'un matériau de traitement dans le récipient de lit fluidisé (3), et
- une unité de commande (12) pour le réglage des conditions de processus à l'intérieur de chaque unité de granulation (2).

2. Installation de lit fluidisé (1) selon la revendication 1, **caractérisée par** une conduite d'entrée (9) par l'intermédiaire de laquelle les entrées (4) de toutes les unités de granulation (2) sont reliées.

3. Installation de lit fluidisé (1) selon l'une des revendications précédentes, **caractérisée par** une conduite de sortie (10) par l'intermédiaire de laquelle les sorties (5) de toutes les unités de granulation (2) sont reliées.

4. Installation de lit fluidisé (1) selon l'une des revendications précédentes, **caractérisée par** un module de nettoyage (11) relié fonctionnellement à chaque unité de granulation (2) et avec lequel chaque unité de granulation (2) peut être nettoyée indépendamment de toute autre unité de granulation (2).

5. Installation de lit fluidisé (1) selon la revendication 4, **caractérisée en ce que** le module de nettoyage (11) est conçu pour effectuer un nettoyage avec retouche manuelle ou un nettoyage sans retouche manuelle.

6. Installation de lit fluidisé (1) selon l'une des revendications précédentes, **caractérisée par** au moins une entrée de module (13), dans laquelle l'entrée de module (13) est reliée aux entrées (4) de toutes les unités de granulation (2), et dans laquelle l'entrée de module (13) peut être commandée par l'unité de commande (12).

7. Installation de lit fluidisé (1) selon la revendication 6, **caractérisée en ce que** l'entrée de module (13) est conçue comme un module de distribution central (18) et/ou un module tampon et/ou une soupape de commutation et/ou un aiguillage et/ou une conduite circulaire.

8. Installation de lit fluidisé (1) selon l'une des revendications précédentes, **caractérisée par** au moins une sortie de module (14), dans laquelle la sortie de module (14) est reliée aux sorties (5) de toutes les unités de granulation (2), et dans laquelle la sortie de module (14) peut être commandée par l'unité de commande (12).

9. Installation de lit fluidisé (1) selon l'une des revendications précédentes, **caractérisée en ce que** l'au moins une buse d'injection (8) est disposée dans le fond (15) et/ou dans l'élément de recouvrement (16) et/ou dans la paroi latérale (17) de l'unité de granulation (2).

10. Procédé pour la production semi-continue de granulés pharmaceutiques dans une installation de lit fluidisé (1) selon la revendication 1, **caractérisé en ce que** des unités de granulation (2) de l'installation de lit fluidisé (1) fonctionnent et/ou sont nettoyées indépendamment les unes des autres.
